Europäisches Patentamt

European Patent Office

Office européen des brevets

(19)

(11) Veröffentlichungsnummer: **0 108 999 B1**

(12) **EUROPÄISCHE PATENTSCHRIFT**

(45) Veröffentlichungstag der Patentschrift:
06.08.86

(21) Anmeldenummer: 83110909.5

(22) Anmeldetag: 02.11.83

(51) Int. Cl.⁴: **C 07 C 69/33, C 07 C 67/08, C 07 C 69/52**

(54) Verfahren zur Herstellung von Carbonsäureestern von Hexiten.

(30) Priorität: 05.11.82 DE 3240892

(43) Veröffentlichungstag der Anmeldung:
23.05.84 Patentblatt 84/21

(45) Bekanntmachung des Hinweises auf die Patenterteilung:
06.08.86 Patentblatt 86/32

(84) Benannte Vertragsstaaten:
BE CH DE FR GB IT LI NL SE

(56) Entgegenhaltungen:
DE - A - 1 200 795
DE - A - 2 423 278

(73) Patentinhaber: HOECHST AKTIENGESELLSCHAFT, Postfach 80 03 20, D-6230 Frankfurt am Main 80 (DE)

(72) Erfinder: Stühler, Herbert, Dr., Hochfellnstrasse 12, D-8269 Burgkirchen (DE)

## Beschreibung

Die Erfindung betrifft ein Verfahren zur Herstellung von Carbonsäureestern von Hexiten durch Umsetzung von Hexiten mit Carbonsäuren in Gegenwart von alkalischen Katalysatoren und einer Inertgasatmosphäre unter Rühren und unter Entfernung des bei der Veresterung sich bildenden Wassers, wobei die erhaltenen Ester im wesentlichen frei sind von Anhydroverbindungen und eine gute Farbqualität besitzen.

Bei der Herstellung von Carbonsäureestern von Hexiten geht es insbesondere darum, die Bildung der weniger hydrophilen Anhydrohexite und Anhydrohexit-Carbonsäureester zu vermeiden. Diese unerwünschten Anhydroverbindungen, beispielsweise Monohydrosorbit (Sorbitan), Dianhydrosorbit (Isosorbit) und die entsprechenden Carbonsäureester, bilden sich bekanntlich durch Dehydratisierungsreaktionen, wobei intramolekular ein mol oder mehrere mol Wasser abgespalten werden. Darüber hinaus geht es auch darum, eine gute Farbqualität zu erreichen.

Wegen der grossen Bedeutung der Carbonsäureester von Hexiten sind schon zahlreiche Versuche unternommen worden, um ein Herstellungsverfahren für diese Ester zu finden, mit dem möglichst wenig Anhydroverbindungen gebildet werden und eine gute Farbqualität erreicht wird.

In einer Reihe von Patentschriften, beispielsweise in den US-Patentschriften 2 997 492 und 2 997 493, wird empfohlen, die Carbonsäureester von Hexiten mit Hilfe von Umesterungsverfahren herzustellen. Damit werden relativ wenig gefärbte Ester erhalten, die zudem im wesentlichen frei sind von Anhydrogruppen. Bei diesen bekannten Umesterungsverfahren müssen jedoch bestimmte Carbonsäureester und spezielle Lösungsmittel eingesetzt werden.

In weiteren zahlreichen Patentschriften, beispielsweise in den US-Patentschriften 1 959 930 und 3 579 547, wird die Bildung von Anhydrogruppen und von stark gefärbten Nebenprodukten dadurch zu verhindern versucht, dass zur Veresterung der Hexite Fettsäurechloride, Fettsäureanhydride und dergleichen eingesetzt werden, oder dass nicht von Hexiten direkt, sondern von Hexitderivaten, d.h. von Hexiten mit Schutzgruppen, ausgegangen wird.

Es sind schliesslich auch schon Verfahren beschrieben worden, bei denen in vorteilhafter Weise direkt von Carbonsäure und Hexit ausgegangen werden kann. Auch all diese, beispielsweise aus der britischen Patentschrift 872 507 und der deutschen Offenlegungsschrift 2 423 278, bekannten Verfahren zur Herstellung von Carbonsäureestern von Hexiten mit guter Farbqualität und einem relativ geringen Anteil von Anhydroverbindungen lassen aber auch noch zu wünschen übrig, weil spezielle Lösungsmittel, lange Reaktionszeiten, relativ hohe Katalysatormengen und dergleichen erforderlich sind.

Bei dem in der britischen Patentschrift 872 507 beschriebenen Verfahren muss zur Umesterung von Carbonsäure und Hexit, die bei einer Temperatur von etwa 97°C vorgenommen wird, ein spezielles Lösungsmittel, nämlich Dimethylformamid eingesetzt werden.

Bei dem in der deutschen Offenlegungsschrift 2 423 278 beschriebenen Verfahren wird Hexit mit Carbonsäure (Fettsäure) in Gegenwart von mindestens 10 Gew.-% Fettsäureseife als Katalysator und in einer Inertgasatomosphäre unter Rühren und unter Entfernung des bei der Veresterung sich bildenden Wassers bei einer Temperatur von 100 bis 190°C umgesetzt (verestert). Nachteilig ist dabei, dass eine relativ lange Reaktionsdauer erforderlich ist. Auch ist die notwendige Katalysatormenge relativ hoch.

Aufgabe der Erfindung ist es daher, ein Verfahren zur Herstellung von Carbonsäureestern von Hexiten zu finden, bei dem direkt von Carbonsäure und Hexit ausgegangen werden kann und keine speziellen Lösungsmittel benötigt werden, das die Carbonsäureester mit einer guten Farbqualität und mit einem nur geringen Anteil von Anhydroverbindungen liefert und das darüber hinaus auch noch weitere Vorteile aufweist, insbesondere den Vorteil einer kurzen Reaktionszeit.

Das erfindungsgemässe Verfahren zur Herstellung von Carbonsäureestern von Hexiten durch Umsetzung von Hexiten mit Carbonsäuren in Gegenwart von alkalischen Katalysatoren und einer Inertgasatmosphäre unter Rühren und unter Entfernung des bei der Veresterung sich bildenden Wassers ist dadurch gekennzeichnet, dass man

a) Hexit und Carbonsäure im Molverhältnis von 1:0,3 bis 1:2,3 einsetzt,

b) den alkalischen Katalysator in einer Menge von 0,01 bis 1 Gew.-%, bezogen auf die Menge von Hexit, einsetzt,

c) bei einer Temperatur von 210 bis 250°C umsetzt, wobei in einer Zeit von höchstens 45 Minuten auf diese Temperatur erhitzt wird,

d) die Umsetzung so lange durchgeführt, bis eine Säurezahl von 2 bis 15 erreicht ist, und

e) nach Erreichung dieser Säurezahl in einer Zeit von höchstens 40 Minuten so abkühlt, das danach die Temperatur des Reaktionsproduktes höchstens 145°C beträgt.

Die Carbonsäuren, die beim erfindungsgemässen Verfahren eingesetzt werden, sind die aliphatischen, geradkettigen oder verzweigten, vorzugsweise geradkettigen, und gesättigten oder ungesättigten (vorzugsweise ein- bis dreifach ungesättigten) Monocarbonsäuren.

Bevorzugte Carbonsäuren sind die Fettsäuren, zweckmässigerweise solche mit 6 bis 30 C-Atomen, vorzugsweise mit 8 bis 22 C-Atomen.

Es können auch Gemische von Carbonsäuren, vorzugsweise Fettsäuregemische, eingesetzt werden.

Vorteilhafte Carbonsäuren sind beispielsweise die Hexansäure (Capronsäure), Octansäure (Caprylsäure), Decansäure (Caprinsäure), Dodecansäure (Laurinsäure), Tetradecansäure (Myristinsäure), Hexadecansäure (Palmitinsäure), Octadecansäure (Stearinsäure), Eicosansäure (Arachinsäure), Docosansäure (Behensäure), Dodecensäure (Lauroleinsäure), Tetradecensäure (Myristoleinsäure), Hexadecensäure (Palmitoleinsäure), Octadecensäure (Ölsäure), 12-Oxy-octadecensäure (Ricinolsäure), Octadecadiensäure (Linolsäure) und Octadecatriensäure (Linolensäure) sowie die Cocosnussölsäure, Talgfettsäure und Palmkernfettsäure.

Die Cocosfettsäure weist in der Regel die folgende typische Kettenverteilung in Gewichtsprozent auf: gesättigt: $C_6$ 0,5%, $C_8$ 8%, $C_{10}$ 7%, $C_{12}$ 48%, $C_{14}$ 17%, $C_{16}$ 9% und $C_{18}$ 2%; ungesättigt: $C_{16}$ (mit einer Doppelbindung) 0,2%, $C_{18}$ (mit einer Doppelbindung) 7% und $C_{18}$ (mit 2 Doppelbindungen) 1,3%. Die Talgfettsäure weist in der Regel die folgende typische Kettenverteilung auf: gesättigt: $C_{12}$ 0,5%, $C_{14}$ 3%, $C_{15}$ 1%, $C_{16}$ 25%, $C_{17}$ 2%, $C_{18}$ 19% und $C_{20}$ 1%; ungesättigt: $C_{14}$ (mit einer Doppelbindung) 0,5%, $C_{16}$ (mit einer Doppelbindung) 3%, $C_{18}$ (mit einer Doppelbindung) 40%, $C_{18}$ (mit zwei Doppelbindungen) 4% und $C_{18}$ (mit drei Doppelbindungen) 1%.

Die Palmkernfettsäure weist in der Regel die folgende typische Kettenverteilung in Gewichtsprozent auf: gesättigt: $C_6$ 0,5%, $C_8$ 4%, $C_{10}$ 5%, $C_{12}$ 50%, $C_{14}$ 15%, $C_{16}$ 7% und $C_{18}$ 2%; ungesättigt: $C_{16}$ (mit einer Doppelbindung) 0,5%, $C_{18}$ (mit einer Doppelbindung) 15% und $C_{18}$ (mit zwei Doppelbindungen) 1%. Die Carbonsäuren werden in der Regel als solche, das heisst ohne jegliche Lösungsmittel, eingesetzt.

Die Hexite, die beim erfindungsgemässen Verfahren eingesetzt werden, sind die sechswertigen gesättigten und geradkettigen aliphatischen Alkohole mit 6 Kohlenstoffatomen, wie Sorbit, Mannit, Dulcit, Idit, Talit und Alit, und deren Ethoxylate und Propoxylate mit 1 bis 10, vorzugsweise 1 bis 5 Ethylenoxid-Einheiten, beziehungsweise 1 bis 5, vorzugsweise 1 bis 3 Propylenoxid-Einheiten, pro mol Hexit. Bevorzugte Hexite sind Sorbit, Mannit und Dulcit und deren Ethoxylate und Propoxylate mit 1 bis 5 Ethylenoxid-Einheiten beziehungsweise 1 bis 3 Propylenoxid-Einheiten pro mol Hexit.

So wie die Carbonsäuren werden auch die Hexite in der Regel in jener Form verwendet, in der sie im allgemeinen vorliegen. Mannit und Dulcit sind beispielsweise in fester Form erhältlich und werden daher als solche eingesetzt. Sorbit ist entweder fest oder als wässrige Lösung (Sirup) erhältlich. Er kann in beiden Formen verwendet werden. Im Falle der Verwendung von wässrigen Lösungen wird das Lösungswasser bereits beim Erhitzen auf die angegebene Reaktionstemperatur ausgetragen.

Das Molverhältnis von Hexit zu Carbonsäure beträgt erfindungsgemäss 1:0,3 bis 1:2,3, vorzugsweise 1:0,5 bis 1:2.

Bei der erfindungsgemässen Umsetzung werden alkalische Katalysatoren eingesetzt. Die Menge an Katalysator beträgt 0,01 bis 1 Gew.-%, vorzugsweise 0,1 bis 0,5 Gew.-%, bezogen auf das Gewicht von Hexit.

Geeignete alkalische (basische) Katalysatoren sind beispielsweise Alkalihydroxide, Alkalicarbonate, Alkalialkoholate mit einer Alkylgruppe mit 1 bis 4 Kohlenstoffatomen und/oder Alkalioxide. Bevorzugt sind, einzeln oder in Mischung, Alkalihydroxide, wie Ätzkali und Ätznatron, Alkalicarbonate, wie Kalium- und Natriumcarbonat, und Alkalialkoholate mit einer Alkylgruppe mit 1 bis 2 Kohlenstoffatomen, wie Kalium- und Natriummethylat.

Beim erfindungsgemässen Verfahren wird die Veresterung von Hexit mit Carbonsäure bei einer Temperatur von 210 bis 250°C, vorzugsweise 220 bis 240°C, vorgenommen, wobei das Aufheizen auf die erfindungsgemässe Reaktionstemperatur rasch erfolgen soll, vorzugsweise in einer Zeit von höchstens 45 Minuten. Die Aufheizzeit beträgt im allgemeinen, je nach Grösse des Ansatzes, 20 bis 40 Minuten. Im Falle der Anwesenheit von Wasser als Lösungsmittel, beispielsweise beim Einsatz der oben erwähnten wässrigen Sorbitlösung, destilliert beim Aufheizen zunächst bei etwa 100°C das Lösungswasser ab. Es ist klar, dass die Zeit für das Abdestillieren dieses Wassers in der angegebenen Aufheizzeit nicht eingeschlossen ist. Im Falle der Anwesenheit von Wasser als Lösungsmittel bezieht sich also die angegebene Aufheizzeit im wesentlichen auf die Zeit, die für das Erhitzen von etwa 100°C (nachdem das Lösungswasser abdestilliert ist) auf die erfindungsgemässe Reaktionstemperatur höchstens benötigt werden soll.

Das rasche Erhitzen auf die Reaktionstemperatur kann mit den hierfür bekannten Hilfsmitteln, beispielsweise mit Heizpilz, Heissölkreislauf und dergleichen, erreicht werden.

Die Mischung aus Hexit, Carbonsäure und Katalysator wird so lange bei der erfindungsgemässen Reaktionstemperatur gehalten, bis die Säurezahl der Mischung 2 bis 15, vorzugsweise 4 bis 10, beträgt. Dies erfordert in der Regel eine Reaktionszeit von 10 bis 60 Minuten. Nachdem die Säurezahl erreicht ist, wird rasch, vorzugsweise in einer Zeit von höchstens 40 Minuten, auf mindestens 145°C abgekühlt. Die Temperatur, auf die das Reaktionsprodukt rasch abgekühlt wird, beträgt vorzugsweise 120 bis 20°C. Die Abkühlzeit liegt, je nach Grösse des Ansatzes, Höhe der Reaktionstemperatur und Kühlweise, im allgemeinen bei 5 bis 30 Minuten. Das rasche Abkühlen kann mit den hierfür bekannten Hilfsmitteln, beispielsweise mit einem Kältebad, Kühlmittelkreislauf und dergleichen, erreicht werden.

Die erfindungsgemässe Umsetzung wird unter Rühren und unter Inertgasatmosphäre durchgeführt. Als Inertgase kommen solche Gase in Betracht, die mit den Ausgangssubstanzen und dem Reaktionsprodukt keine Reaktion eingehen. Bevorzugt sind Stickstoff, Kohlendioxid und/oder Edelgase. Die Inertgas-Atmosphäre wird beim erfindungsgemässen Verfahren vorzugsweise dadurch erzeugt, dass ein Inertgasstrom über oder durch die Reaktionsmischung geleitet wird. Er beträgt im allgemeinen pro Kilogramm Reaktionsmischung 5 bis 30 l pro Stunde, vorzugsweise 10 bis 20 l pro Stunde.

Die erfindungsgemässe Umsetzung wird in der Regel bei Atmosphärendruck (Normaldruck) durchgeführt. Sie kann auch mit Unterdruck durchgeführt werden. Das Vakuum kann in weiten Grenzen variieren. Seine Grenze liegt lediglich dort, wo auch Reaktionskomponenten oder Reaktionsprodukte ausgetragen werden können. Im Falle von Unterdruck liegt zweckmässigerweise ein Druck von 100 bis 5000 Pa, vorzugsweise von 2000 bis 4000 Pa, vor.

Die Umsetzung kann kontinuierlich, beispielsweise in einer Rohrschlange, oder diskontinuierlich, beispielsweise chargenweise in einem Reaktionsgefäss, durchgeführt werden.

Das bei der Veresterung entstehende Wasser wird laufend entfernt. Die Wasserentfernung aus dem Reaktionssystem erfolgt bereits aufgrund der hohen Reaktionstemperatur. Der Inertgasstrom und der gegebenenfalls vorhandene Unterdruck bewirkt ebenfalls die Austragung von vorhandenem Wasser.

Das Reaktionsprodukt des erfindungsgemässen Verfahrens, das nach Erreichung der angegebenen Säurezahl und nach Abkühlen vorliegt, stellt eine mehr oder weniger viskose Flüssigkeit oder einen Feststoff dar und besteht in der Regel aus einer Mischung von einzelnen Hexitcarbonsäureestern. Es enthält je nach Reaktionsbedingungen und Molverhältnis von Hexit zu Carbonsäure hauptsächlich Hexitmono- und Hexitdicarbonsäureester.

Da das Reaktionsprodukt bereits als solches vielseitig verwendbar ist, erübrigt sich im allgemeinen eine Isolierung von einzelnen Estern oder von Gruppen von Estern. Es kann aber zweckmässig sein, im Produkt befindliche Katalysatoren und gegebenenfalls nicht-umgesetzten Hexit und nicht-umgesetzte Carbonsäure, beispielsweise durch Waschen mit wässrigen Lösungen von Salzen, wie Natriumchlorid und Natriunsulfat, zu entfernen.

Mit dem erfindungsgemässen Verfahren werden Carbonsäureester von Hexiten erhalten, die im wesentlichen frei sind von Anhydroverbindungen und die nur relativ wenig gefärbt sind. Sie weisen im allgemeinen keine stärkere Färbung auf als die Eigenfarbe der eingesetzten Carbonsäure.

Falls Carbonsäureester mit einer besonders guten Farbqualität gewünscht werden, kann das Reaktionsprodukt einer diesbezüglichen Behandlung, vorzugsweise einer Bleichung unterworfen werden. Nach einer bevorzugten Ausführung wird die Bleichung in der Weise durchgeführt, dass das Reaktionsprodukt bei einer Temperatur von 50 bis 120°C, vorzugsweise 70 bis 100°C, mit etwa 0,5 bis 5 Gew.-%, vorzugsweise etwa 1 bis 3 Gew.-%, bezogen auf das Gewicht des Reaktionsproduktes, einer etwa 35 gew.-%igen wässrigen Wasserstoffperoxid-Lösung versetzt und etwa 10 bis 30 Minuten lang bei der genannten Temperatur gerührt wird (es tritt keine weitere Aufhellung des Reaktionsproduktes mehr ein), worauf das durch die Wasserstoffperoxid-Behandlung eingebrachte Wasser abdestilliert und der Rückstand (das Reaktionsprodukt) zweckmässigerweise noch filtriert oder abgenutscht wird. Das Abdestillieren des Wassers erfolgt vorzugsweise bei einer Temperatur von 60 bis 120°C und einem Vakuum von 500 bis 2000 Pa.

Die mit dem erfindungsgemässen Verfahren hergestellten Carbonsäureester von Hexiten sind wertvolle oberflächenaktive Substanzen, insbesondere Emulgatoren und Gleitmittel. Sie werden vorteilhaft beispielsweise zur Herstellung von Wasser-in-Öl- oder Öl-in-Wasser-Emulsionen, bei der Formulierung von kosmetischen Präparaten, in der Waschmittelindustrie sowie als Gleitmittel in der Kunststoffverarbeitung eingesetzt. Bei diesen oder anderen Verwendungsarten kann es unter Umständen zweckmässig sein, die oberflächenaktiven Eigenschaften der erfindungsgemäss hergestellten Carbonsäureester zu variieren, beispielsweise durch Ethoxylierung und/oder Propoxylierung.

Die Erfindung wird nun an Beispielen noch näher erläutert:

*Beispiele 1 bis 10*

In einem mit Thermometer, Rührer, Wasserabscheidevorrichtung und einer Vorrichtung zum Durchleiten eines Inertgases ausgestatteten 2-Liter-Reaktionsgefäss wurden jeweils Carbonsäure, Hexit und alkalischer Katalysator vorgelegt. Das Reaktionsgemisch wurde, gegebenenfalls nach Schmelzen, unter Rühren und Durchleiten von etwa 20 l Stickstoff pro Stunde und pro kg Reaktionsgemisch mit Hilfe eines Heizpilzes rasch auf die erfindungsgemässe Temperatur erhitzt und bei dieser Temperatur unter Rühren gehalten. Das während der Umsetzung (bei Atmosphärendruck) entstehende Wasser (aus der Veresterungsreaktion) wurde gleichzeitig über die Wasserabscheidevorrichtung abgeführt. Nach Erreichen der erfindungsgemäss angestrebten Säurezahl wurde mit Hilfe von Kühlluft und/oder Kältewasserbad abgekühlt, so dass das Reaktionsprodukt rasch eine Temperatur (Abkühltemperatur) annahm, die unterhalb der erfindungsgemässen Temperaturgrenze lag.

Zur Charakterisierung des erhaltenen Produktes wurde jeweils die Hydroxylzahl (OH-Zahl) bestimmt.

Die Bestimmung der Säurezahl und der OH-Zahl erfolgte nach den «Einheitsmethoden der deutschen Gesellschaft für Fettwissenschaft».

In der nachstehenden Tabelle ist die Durchführung der Beispiele 1 bis 10 im einzelnen angegeben. Die Tabelle enthält auch die gemessenen OH-Zahl-Werte sowie die berechneten (theoretischen) OH-Zahl-Werte.

TABELLE

| Bei-spiele | mol Hexit | | mol Fettsäure | Kataly-sator und Menge (%) | Tempe-ratur (°C) | Auf-heiz-zeit (min) | Säure-zahl | Reak-tions-zeit (min) | Ab-kühl-zeit (min) | Abkühl-tempe-ratur (°C) | OH-Zahl gemes-sen | OH-Zahl berech-net |
|---|---|---|---|---|---|---|---|---|---|---|---|---|
| 1 | 2,5 | Sorbit | 2,5 Kokos-fettsäure | NaOH 0,4 | 240 | 35 | 4 | 15 | 20 | 140 | 678 | 768 |
| 2 | 1,5 | Sorbit | 3 Kokos-fettsäure | NaOH 0,4 | 240 | 30 | 10 | 10 | 25 | 110 | 346 | 409 |
| 3 | 1,5 | Mannit | 3 Ölsäure | NaOH 0,4 | 240 | 40 | 13 | 10 | 5 | 145 | 258 | 316 |

TABELLE (Fortsetzung)

| Bei-spiele | mol Hexit | | mol Fettsäure | Kataly-sator und Menge (%) | Tempe-ratur (°C) | Auf-heiz-zeit (min) | Säure-zahl | Reak-tions-zeit (min) | Ab-kühl-zeit (min) | Abkühl-tempe-ratur (°C) | OH-Zahl gemes-sen | OH-Zahl berech-net |
|---|---|---|---|---|---|---|---|---|---|---|---|---|
| 4 | 2 | Dulcit | 2 Isostea-rinsäure | NaOH 0,4 | 240 | 35 | 5 | 40 | 10 | 100 | 419 | 594 |
| 5 | 3 | Sorbit | 1,5 Stea-rinsäure | NaOH 0,4 | 220 | 30 | 15 | 55 | 10 | 80 | 840 | 967 |
| 6 | 2,5 | Sorbit + 2 EO | 0,75 Kokos-fettsäure | KOH 0,5 | 210 | 25 | 2 | 20 | 35 | 60 | 585 | 594 |
| 7 | 2 | Mannit + 4 EO | 1 Kokos-fettsäure | $Na_2CO_3$ 0,1 | 220 | 35 | 2 | 20 | 40 | 145 | 515 | 500 |
| 8 | 2 | Sorbit + 1 PyO | 2 Kokos-fettsäure | $NaOCH_3$ 0,08 | 250 | 45 | 3 | 10 | 5 | 130 | 558 | 665 |
| 9 | 1 | Sorbit + 3 PyO | 2,3 Kokos-fettsäure | NaOH 0,2 | 240 | 40 | 6 | 45 | 15 | 65 | 260 | 268 |
| 10 | 2 | Dulcit + 1 EO | 2 Stearin-säure | NaOH 0,2 | 240 | 40 | 3 | 25 | 40 | 30 | 524 | 565 |

EO  =  Ethylenoxid
PyO =  Propylenoxid

## Patentansprüche

1. Verfahren zur Herstellung von Carbonsäureestern von Hexiten durch Umsetzung von Hexiten mit Carbonsäuren in Gegenwart von alkalischen Katalysatoren und einer Inertgasatmosphäre unter Rühren und unter Entfernung des bei der Veresterung sich bildenden Wassers, dadurch gekennzeichnet, dass man
a) Hexit und Carbonsäure im Molverhältnis von 1:0,3 bis 1:2,3 einsetzt,
b) den alkalischen Katalysator in einer Menge von 0,01 bis 1 Gew.-%, bezogen auf die Menge von Hexit, einsetzt,
c) bei einer Temperatur von 210 bis 250°C umsetzt, wobei in einer Zeit von höchstens 45 Minuten auf diese Temperatur erhitzt wird,
d) die Umsetzung so lange durchgeführt, bis eine Säurezahl von 2 bis 15 erreicht ist, und
e) nach Erreichung dieser Säurezahl in einer Zeit von höchstens 40 Minuten so abkühlt, dass danach die Temperatur des Reaktionsproduktes höchstens 145°C beträgt.

2. Verfahren nach Anspruch 1, dadurch gekennzeichnet, dass man
a) Hexit und Carbonsäure im Molverhältnis von 1:0,5 bis 1:2 einsetzt,
b) den alkalischen Katalysator in einer Menge von 0,1 bis 0,5 Gew.-% einsetzt,
c) bei einer Temperatur von 220 bis 240°C umsetzt,
d) bis zu einer Säurezahl von 4 bis 10 umsetzt, und
e) auf eine Temperatur des Reaktionsproduktes von 120 bis 20°C abkühlt.

3. Verfahren nach Anspruch 2, dadurch gekennzeichnet, dass man in einer Zeit von 20 bis 40 Minuten erhitzt und in einer Zeit von 5 bis 30 Minuten abkühlt.

4. Verfahren nach einem der Ansprüche 1 bis 3, dadurch gekennzeichnet, dass man als Hexit Sorbit, Mannit, Dulcit oder deren Ethoxylate oder Propoxylate mit 1 bis 5 Ethylenoxid-Einheiten beziehungsweise 1 bis 3 Propylenoxid-Einheiten pro mol Hexit und als Carbonsäure eine Fettsäure einsetzt.

## Claims

1. A process for preparing carboxylic acid esters of hexitols by reacting hexitols with carboxylic acids in the presence of alkaline catalysts and an inert gas atmosphere while stirring and while removing the water formed in the course of the esterification, which comprises
a) using hexitol and carboxylic acid in a molar ratio of 1:0,3 to 1:2,3,
b) using the alkaline catalyst in an amount of 0,01 to 1% by weight, relative to the amount of hexitol,
c) carrying out the reaction at a temperature of 210 to 250°C, heating up to this temperature within a period of at most 45 minutes,
d) continuing the reaction until an acid number of 2 to 15 has been reached, and
e) cooling down the reaction product after said acid number has been reached within a period of at most 40 minutes to a temperature of at most 145°C.

2. A process as claimed in claim 1, wherein
a) hexitol and carboxylic acid are used in a molar ratio of 1:0,5 to 1:2,
b) the alkaline catalyst is used in an amount of 0.1 to 0.5% by weight,
c) the reaction is carried out at a temperature of 220 to 240°C,
d) the reaction is carried out until an acid number of 4 to 10 has been reached, and

e) the reaction product is cooled down to a temperature of 120 to 20°C.

3. A process as claimed in claim 2, wherein the reaction mixture is heated to the reaction temperature within a period of 20 to 40 minutes, and the reaction product is cooled down within a period of 5 to 30 minutes.

4. A process as claimed in any one of the claims 1 to 3, wherein the hexitol used is sorbitol, mannitol, dulcitol or their ethoxylates or propoxylates having 1 to 5 ethylene oxide units or, respectively, 1 to 3 propylene oxide units per mole of hexitol, and the carboxylic acid used is a fatty acid.

**Revendications**

1. Procédé pour préparer des esters dérivants d'acides carboxyliques et d'hexitols par réaction d'héxitols avec des acides carboxyliques en présence de catalyseurs alcalins, sous un gaz inerte, tout en agitant et tout en éliminant l'eau formée au cours de l'estérification, procédé caractérisé en ce que:
a) on fait réagir l'hexitol et l'acide carboxylique dans un rapport molaire de 1:0,3 à 1:2,3,
b) on utilise le catalyseur alcalin en une quantité de 0,01 à 1% en poids par rapport à la quantité de l'hexitol,
c) on fait réagir à une température de 210 à 250°C,

cette température étant atteinte par un chauffage d'une durée d'au plus 45 minutes,
d) on effectue la réaction jusqu'à ce que l'indice d'acide ait atteint une valeur de 2 à 15 et
e) lorsque cet indice d'acide est atteint on refroidit en un temps d'au plus 40 minutes de telle façon qu'après cela la température du produit réactionnel soit d'au plus 145°C.

2. Procédé selon la revendication 1 caractérisé en ce que:
a) on utilise l'hexitol et l'acide carboxylique dans le rapport molaire de 1:0,5 à 1:2,
b) on utilise le catalyseur alcalin en une quantité de 0,1 à 0,5% en poids,
c) on fait réagir à une température de 220 à 240°C,
d) on fait réagir jusqu'à ce que l'indice d'acide soit de 4 à 10 et
e) on refroidit à une température du produit réactionnel de 120 à 20°C.

3. Procédé selon la revendication 2 caractérisé en ce qu'on chauffe en 20 à 40 minutes et on refroidit en 5 à 30 minutes.

4. Procédé selon l'une quelconque des revendications 1 à 3, caractérisé en ce qu'on utilise, comme hexitol, le sorbitol, le mannitol, le dulcitol ou leurs produits d'éthoxylation ou de propoxylation contenant respectivement de 1 à 5 motifs d'oxyde d'éthylène ou de 1 à 3 motifs d'oxyde de propylène par mole d'hexitol, et, comme acide carboxylique, un acide gras.